# EUROPÄISCHE PATENTANMELDUNG

(11) **EP 3 147 288 A1**
(43) Veröffentlichungstag der Anmeldung: **29.03.2017**
(21) Anmeldenummer: 15186164.8
(22) Anmeldetag: 22.09.2015
(51) Int. Cl.: C07F 9/6574, C07C 45/50

(54) **MONOPHOSPHITE, DIE EIN CYCLODODECANOL AUFWEISEN**

(71) Anmelder: Evonik Degussa GmbH, 45128 Essen (DE)
(72) Erfinder: BÖRNER, Armin, 18059 Rostock (DE); DYBALLA, Katrin Marie, 45657 Recklinghausen (DE); FRANKE, Robert, 45772 Marl (DE); FRIDAG, Dirk, 45721 Haltern am See (DE); GEILEN, Frank, 45721 Haltern am See (DE); HESS, Dieter, 45770 Marl (DE); HÄGER, Harald, 59348 Lüdinghausen (DE); SELENT, Detlef, 18059 Rostock (DE)

(57) **Zusammenfassung**

Die Erfindung betrifft Monophosphite, die ein Cyclododecanol aufweisen, sowie deren Verwendung als Liganden für Hydroformylierungskatalysatoren.

## Beschreibung

Die Erfindung betrifft Monophosphite, die ein Cyclododecanol aufweisen, sowie deren Verwendung als Liganden für Hydroformylierungskatalysatoren.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor PIII. Eine gute Übersicht über den Stand der Hydroformylierung von Olefinen findet sich in B. Cornils, W. A. Herrmann, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Die Hydroformylierung führt, außer im Fall von Ethylen als Edukt, zu einer Mischung isomerer Produkte, nämlich n-Aldehyden (n = lineare Aldehyde) und iso-Aldehyden (i = verzweigte Aldehyde). Neben der Reaktionsgeschwindigkeit ist somit auch die Selektivität bei der Bildung von n- bzw. iso-Produkten ein wichtiger Parameter der Hydroformylierungsreaktion.

Die einfache Verfügbarkeit und damit verbunden die gute Möglichkeit eines großtechnischen Einsatzes ist ein wichtiges Kriterium, da der Herstellungsaufwand und damit verbunden die anfallenden Kosten nur so hoch sein dürfen, dass die Rentabilität des Gesamtprozesses weiterhin gewährleistet ist.

Rhodium-Monophosphit-Komplexe in katalytisch aktiven Zusammensetzungen sind geeignet für die Hydroformylierung von verzweigten Olefinen mit innenständigen Doppelbindungen.

Seit den 1970er Jahren ist die Verwendung von so genannten "bulky phosphites" in der Hydroformylierungbeschrieben (siehe u.a. van Leeuwen et. al, Journal of Catalysis, 2013, 298, 198-205). Diese zeichnen sich durch eine gute Aktivität aus, jedoch ist die n/i-Selektivität für endständig oxierte Verbindungen gering und verbesserungswürdig.

Aus EP 0 155 508 A1 ist die Verwendung von bisarylensubstituierten Monophosphiten bei der rhodiumkatalysierten Hydroformylierung von sterisch gehinderten Olefinen, z. B. Isobuten, bekannt. Hierbei werden jedoch z.T. sehr hohe Rhodiumkonzentrationen verwendet (u.a. 250 ppm), was in Anbetracht des derzeitigen Rhodiumpreises für ein großtechnisches Verfahren inakzeptabel ist und verbessert werden muss.

Für Hydroformylierungsreaktionen ist derzeit Tris(2,4-di-tert-butylphenyl)phosphit (TDTBPP) einer der leistungsstärksten und kommerziell verfügbaren Monophosphitliganden, welcher unter dem Handelsnamen Alkanox 240 erhältlich ist (siehe auch R. Franke, D. Selent, A. Börner, "Applied Hydroformylation", Chem. Rev., 2012, 112, S.5681 Kapitel 3.4.2).

Der vorliegenden Erfindung lag vor diesem Hintergrund die Aufgabe zugrunde, neue Liganden für die Hydroformylierung bereitzustellen, welche eine hohe Ausbeute bei gleichzeitig geringer n/i-Selektivität gewährleisten. Somit soll die Durchführung nichtisomerisierender Hydroformylierungsreaktionen ermöglicht werden.

Die Aufgabe wird gelöst durch eine Verbindung gemäß Formel (I): 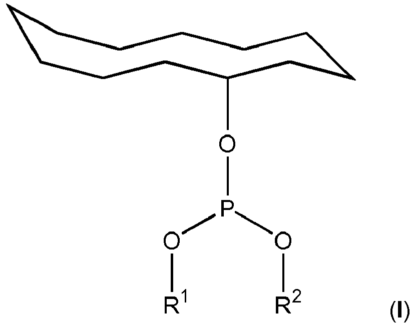 wobei R¹ und R² jeweils eine Cyclododecanylgruppe oder zwei kovalent miteinander verknüpfte (C₆-C₂₀)-Arylgruppen darstellen oder zusammen eine (C₆-C₂₀)-Arylgruppe oder eine (C₁-C₁₂)-Alkylgruppe bilden,
wobei R¹ und R² mit einem oder mehreren Substituenten ausgewählt aus der Gruppe bestehend aus -H, -(C₆-C₂₀)-Aryl, -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl,-CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -COOH, -OH, -SO₃H, -NH₂, und Halogen substituiert sein können.

Die erfindungsgemäßen Phosphite gemäß Formel (I) zeichnen sich dadurch aus, dass sie mindestens ein Cyclododecanyl in Kombination mit weiteren, sterisch anspruchsvollen Substituenten R¹ und R² umfassen. Sie eignen sich in besonderer Weise als Liganden für Hydroformylierungskatalysatoren. Unter Verwendung dieser Liganden lassen sich hohe Ausbeuten von Aldehyden mit einem ausgewogenen n/i-Verhältnis erzielen. Die n/i-Selektivität ist durchweg gering, insbesondere bei der Hydroformylierung von endständigen Olefinen. Diese Vorteile lassen sich außerdem bei einem relativ geringen Verhältnis von Ligand zu katalytisch aktivem Edelmetall, z.B. Rh, erzielen.

Im Rahmen der Erfindung umfasst der Ausdruck -(C₁-C₁₂)-Alkyl geradkettige und verzweigte Alkylgruppen mit 1 bis 12 Kohlenstoffatomen. Vorzugsweise handelt es sich dabei um geradkettige oder verzweigte -(C₁-C₈)-Alkyl- und ganz bevorzugt um geradkettige oder verzweigte -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, -S-(C₁-C₁₂)-Alkyl, -COO-(C₁-C₁₂)-Alkyl, -CONH-(C₁-C₁₂)-Alkyl, -CO-(C₁-C₁₂)-Alkyl, und -N-[(C₁-C₁₂)-Alkyl]₂.

Der Ausdruck -(C₃-C₁₂)-Cycloalkyl umfasst im Sinne der vorliegenden Erfindung zyklische Kohlenwasserstoffreste mit 3 bis 12, insbesondere 5 bis 12 Kohlenstoffatomen. Insbesondere handelt es sich dabei um mono-, bi-odertricyclische Kohlenwasserstoffreste. Dazu zählen Cyclopropyl, Cyclobutyl, Cyclopentyl, Cyclohexyl, Cycloheptyl, Cyclooctyl, Cyclododecyl, Cyclopentadecyl, Norbonyl oder Adamantyl. Unter den Begriff -(C₃-C₁₂)-Cycloalkyl fallen auch alkylsubstituierte Cycloalkylgruppen mit insgesamt 3 bis 12 Kohlenstoffatomen, wie beispielsweise Menthyl.

Der Ausdruck -(C₃-C₁₂)-Heterocycloalkyl umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter-O-, -S-, -N-, -N(=O)-, -C(=O)- oder -S(=O)-. Beispiele für -(C₃-C₁₂)-Heterocycloalkylgruppen Tetrahydrothiophenyl, Tetrahydrofuryl, Tetrahydropyranyl und Dioxanyl.

Der Ausdruck -(C₆-C₂₀)-Aryl umfasst im Sinne der vorliegenden Erfindung mono- oder polycyclische aromatische Kohlenwasserstoffreste mit 6 bis 20 Kohlenstoffatomen. Geeignete Arylgruppen sind beispielsweise Phenyl, Naphthyl, Indenyl, Fluroenyl, Anthracenyl, Phenanthrenyl, Naphthacenyl, Chrysenyl, Pyrenyl, und Coronenyl.

Bei den genannten Halogen-Substituenten handelt es sich bevorzugt um Fluor oder Chlor.

Bevorzugte Substituenten sind ausgewählt aus der Gruppe bestehend aus -H, -(C₆-C₂₀)-Aryl, -(C₁-C₁₂)-Alkyl, -(C₃-C₁₂)-Cycloalkyl und -O-(C₁-C₁₂)-Alkyl.

Für den Fall, dass es sich bei R¹ und R² jeweils um Cyclododecanylgruppen handelt, sind diese bevorzugt unsubstituiert.

Für den Fall, dass R¹ und R² zusammen zwei kovalent miteinander verknüpfte (C₆-C₂₀)-Arylgruppen bilden, handelt es sich hierbei bevorzugt um Diphenyl, Dinaphthyl, Dianthracenyl, Diphenanthryl oder Phenylnaphthyl. In jedem Fall sind die beiden verknüpfte (C₆-C₂₀)-Arylgruppen über eine einzige kovalente C-C-Bindung miteinander verknüpft. Bezogen auf diese Bindung können die beiden (C₆-C₂₀)-Arylgruppen S- oder R-Konfiguration aufweisen und auch als Racemat vorliegen. Falls R¹ und R² zusammen jeweils zwei gleiche, kovalent miteinander verknüpfte (C₆-C₂₀)-Arylgruppen bilden, wie z.B. in Diphenyl und Dinaphthyl, können diese Arylgruppen symmetrisch oder asymmetrisch substituiert sein. Ein asymmetrisches Substitutionsmuster ist hierbei bevorzugt.

Für den Fall, dass R¹ und R² zusammen eine (C₆-C₂₀)-Arylgruppe bilden, handelt es sich hierbei bevorzugt um eine Phenyl-, Naphthyl- oder Anthracylgruppe, besonders bevorzugt um eine Phenylgruppe. Die Arylgruppe kann symmetrisch oder asymmetrisch substituiert sein. Ein symmetrisches Substitutionsmuster ist hierbei bevorzugt. Am meisten bevorzugt sind hierbei unsubstituierte Arylgruppen.

Für den Fall, dass R¹ und R² zusammen eine (C₁-C₁₂)-Alkylgruppe bilden, handelt es sich hierbei bevorzugt um eine (C₁-C₆)-Alkylgruppe, besonders bevorzugt um eine Ethylgruppe. In einer bevorzugten Ausführungsform sind R¹ und R² mit mindestens einer -(C₆-C₂₀)-Arylgruppe substiuiert, falls R¹ und R² zusammen eine (C₁-C₁₂)-Alkylgruppe bilden. Die Alkylgruppe kann symmetrisch oder asymmetrisch substituiert sein. Ein symmetrisches Substitutionsmuster ist hierbei bevorzugt.

In einer Ausführungsform stellen R¹ und R² jeweils eine Cyclododecanylgruppe oder zwei kovalent miteinander verknüpfte Phenyl- oder Naphthylgruppen dar oder bilden zusammen eine Phenyl- oder eine Ethylgruppe, wobei R¹ und R² mit einem oder mehreren Substituenten ausgewählt aus der Gruppe bestehend aus -H, -(C₆-C₂₀)-Aryl, -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₃-C₁₂)-Cycloalkyl,-S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl,-CONH-(C₁-C₁₂)-Alkyl, -CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -COOH, -OH, -SO₃H, -NH₂, und Halogen substituiert sein können.
Bevorzugt sind R¹ und R² mit Substituenten ausgewählt aus der Gruppe bestehend aus - H, -(C₆-C₂₀)-Aryl, -(C₁-C₁₂)-Alkyl, substituiert.

In einer Ausführungsform ist die Verbindung ausgewählt aus einer der Strukturen gemäß den Formeln **(II)** bis **(VI):** 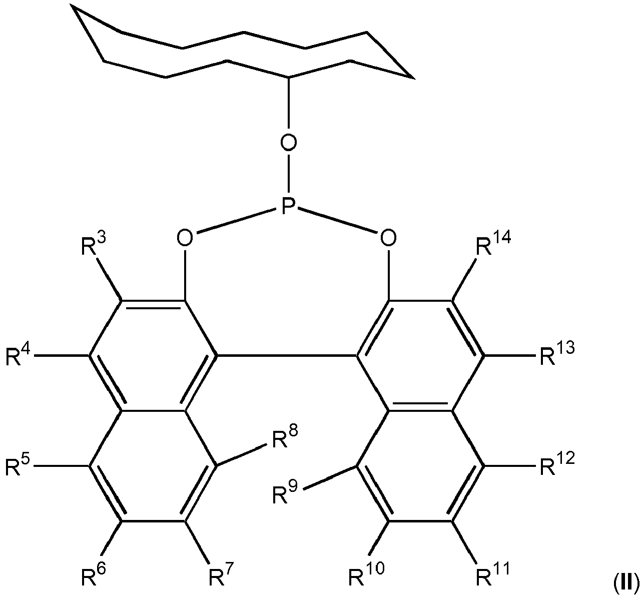 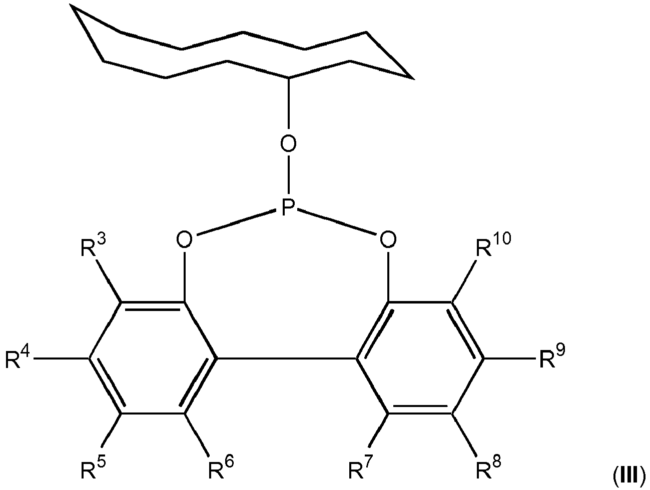 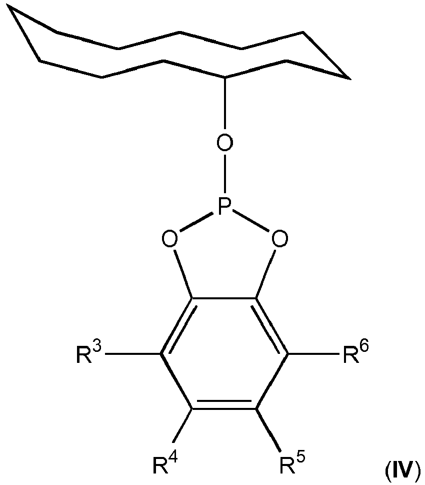 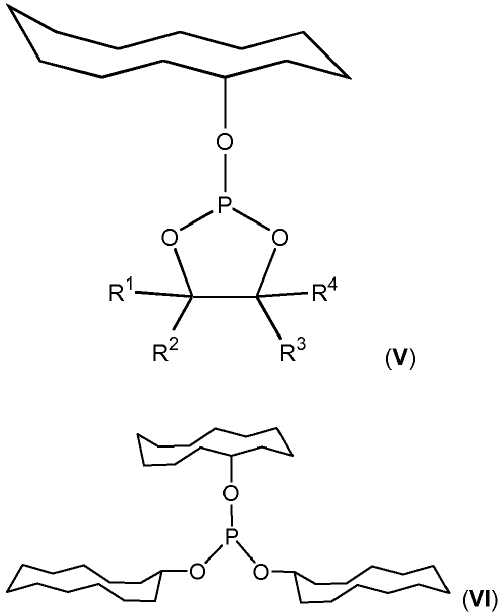 wobei R³ bis R¹⁴ unabhängig voneinander ausgewählt sind aus der Gruppe bestehend aus -H, -(C₆-C₂₀)-Aryl, -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl,-CO-(C₁-C₁₂)-Alkyl, -CO-(C₃-C₁₂)-Cycloalkyl, -N-[(C₁-C₁₂)-Alkyl]₂, -COOH, -OH, -SO₃H,-NH₂, und Halogen.

In einer bevorzugten Ausführungsform sind R³ bis R¹⁴ unabhängig voneinander ausgewählt aus der Gruppe bestehend aus -H, -Phenyl, -(C₁-C₆)-Alkyl, und -O-(C₁-C₆)-Alkyl.

In einer bevorzugten Ausführungsform ist die Verbindung ausgewählt aus einer der Strukturen gemäß den Formeln **(II)** bis **(V),** besonders bevorzugt gemäß den Formeln **(II), (III)** und **(V),** wobei R³ bis R¹⁴ unabhängig voneinander ausgewählt sind aus der Gruppe bestehend aus -H, -(C₆-C₂₀)-Aryl, -(C₁-C₁₂)-Alkyl, -(C₃-C₁₂)-Cycloalkyl , und -O-(C₁-C₁₂)-Alkyl. Bevorzugt sind R³ bis R¹⁴ in dieser Ausführungsform unabhängig voneinander ausgewählt sind aus der Gruppe bestehend aus -H, -Phenyl, -(C₁-C₆)-Alkyl, und -O-(C₁-C₆)-Alkyl.

Im Falle der Struktur gemäß Formel **(II)** sind R³ bis R¹⁴ bevorzugt -H.

Im Falle der Struktur gemäß Formel **(III)** sind R³ bis R¹⁰ bevorzugt ausgewählt aus der Gruppe bestehend aus -H, -(C₁-C₆)-Alkyl, und -O-(C₁-C₆)-Alkyl, besonders bevorzugt aus der Gruppe bestehend aus -H, tert-Butyl, -O-CH₃ und Methyl.

Im Falle der Struktur gemäß Formel **(IV)** sind R³ bis R⁶ bevorzugt -H.

Im Falle der Struktur gemäß Formel **(V)** sind R³ bis R⁴ bevorzugt Phenylgruppen.

In einer Ausführungsform ist die Verbindung ausgewählt aus einer der Strukturen gemäß den Formeln **(1)** bis **(6),** wobei von Formel **(1)** auch die reinen *S*- und *R*-Enantiomere umfasst sind:

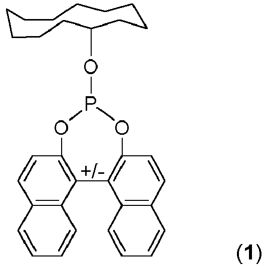

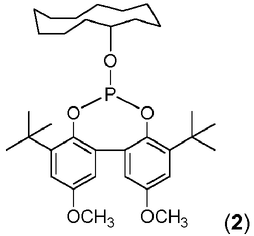

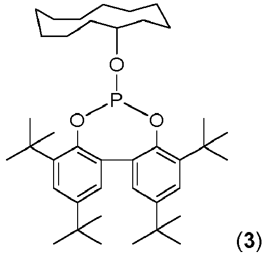

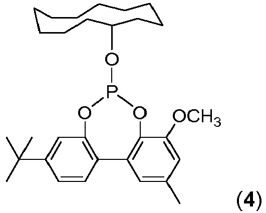

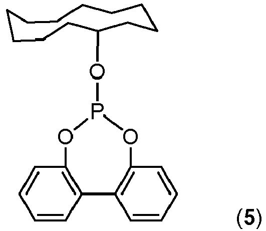

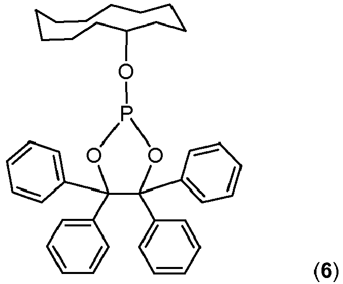

Neben den genannten Verbindungen betrifft die Erfindung auch einen Komplex, welcher die genannte Verbindung und ein Metallatom ausgewählt aus Rh, Ru, Co und Ir umfasst. In einer bevorzugten Ausführungsform ist das Metall Rh.

Die Herstellung derartiger Edelmetallkomplexe ist aus dem Stand der Technik bekannt. Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren betrifft die Erfindung die Verwendung der Verbindung zur Katalyse einer Hydroformylierungsreaktion. Dabei wird die erfindungsgemäße Verbindung bevorzugt als Ligand in einem erfindungsgemäßen Ligand-Metall-Komplex eingesetzt. Ebenso betrifft die Erfindung des beschriebenen Komplexes zur Katalyse einer Hydroformylierungsreaktion. Besonders bevorzugt ist hierbei die Hydroformylierung endständiger Olefine zu Aldehyden.

Die Erfindung betrifft ebenfalls ein Verfahren, bei dem die erfindungsgemäße Verbindung als Ligand in einem Ligand-Metall-Komplex zur Umsetzung eines Olefins zu einem Aldehyd eingesetzt wird.

Das erfindungsgemäße Verfahren umfasst die Verfahrensschritte:
a) Vorlegen eines Olefins,
b) Zugabe eines erfindungsgemäßen Komplexes oder einer erfindungsgemäßen Verbindung und einer Substanz, welche ein Metallatom ausgewählt aus Rh, Ru, Co und Ir aufweist,
c) Zuführen von Wasserstoff und Kohlenstoffmonoxid,
d) Erwärmen des Reaktionsgemisches, wobei das Olefin zu einem Aldehyd umgesetzt wird.

Hierbei können die Verfahrensschritte a) bis c) in beliebiger Reihenfolge erfolgen.

In einer bevorzugten Variante des Verfahrens ist das Metallatom Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten. Vorzugsweise liegt das molare Verhältnis der erfindungsgemäßen Verbindung zum Metallatom im Bereich von 10:1 zu 1:1, bevorzugt 8:1 zu 1:1, besonders bevorzugt 6:1 zu 2:1.

Die Reaktion wird bevorzugt bei einer Temperatur von 80 °C bis 200 °C und einem Druck von 1 bar bis 300 bar durchgeführt. Besonders bevorzugt sind eine Temperatur von 100 °C bis 160 °C und ein Druck von 15 bar bis 250 bar. Besonders bevorzugt sind eine Temperatur von 110 °C bis 130 °C und ein Druck von 30 bar bis 70 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1- oder 2-Penten, 2-Methyl-1-buten, 2-Methyl-2-buten, 3-Methyl-1-buten, 1-, 2- oder 3-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische. Bevorzugt handelt es sich um Monoolefine mit einer endständigen C-C-Doppelbindung.

### Beispiele

Die Erfindung wird anhand der folgenden Ausführungsbeispiele erläutert.

### Allgemeine Arbeitsvorschriften

Alle präparativen Arbeiten erfolgten unter Anwendung der Schlenk-Technik mit Argon als Schutzgas. Toluol und Tetrahydrofuran wurden mit einem Pure Solv MD-7 System gereinigt und bis zur Verwendung unter Argon aufbewahrt. Triethylamin wurde vordem Einsatz unter Argon von Natriumketyl destilliert. Phosphortrichlorid (Aldrich) wurde vor dem Einsatz unter Argon destilliert. Alle präparativen Arbeiten erfolgten in ausgeheizten Gefäßen. Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten, die halbautomatische Säulenchromatografie an einem Teledyne Isco Combiflash Rf+.

### Ligand 1

### rac-4-(Cyclododecyloxy)dinaphtho[2,1-d:1',2'-f][1,3,2]dioxaphosphepin

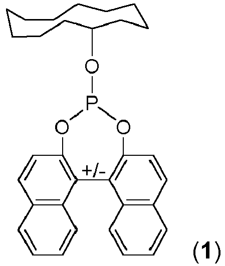

Zu einer auf 0 °C gekühlten Mischung aus Cylododecanol (0,347 g; 1,884 mmol), Triethylamin (2,4 ml) und Toluol (10 ml) wird unter Rühren eine Lösung von *rac*-4-Chlor-dinaphtho[2,1-*d*:1',2'-*f*][1,3,2]dioxaphosphepin (0,661 g; 1,884 mmol) in Toluol (4 ml) getropft. Man lässt über Nacht rühren, filtriert und entfernt das Lösungsmittel im Vakuum. Zum sirupösen Rückstand gibt man Dichlormethan (2 ml), rührt kurz und entfernt das Lösungsmittel im Vakuum. Der erhaltene Feststoff wird in heißem Acetonitril (10 ml) gelöst und die Lösung im Kühlschrank gelagert. Filtration, Waschen mit kaltem Acetonitril (2 ml) und Trocknen bei 25°C/0,1 mbar über 6 h ergibt 0,545 g (1,093 mmol; 58%) Produkt. Elementaranalyse (ber. für C₃₂H₃₅O₃P= 498,599 g/mol): C 77,08 (77,09); H 7,31 (7,07); P 6,02 (6,21)%.
ESI-TOF/HRMS: *m*/*e* 521,22146 (*M*+Na)⁺.
³¹P-NMR (CD₂Cl₂): 150,3 (s) ppm.
¹H-NMR (CD₂Cl₂): 1,33-1,92 (m, 22H); 4,53 (m, 1 H); 7,28-7,51 (m, 7H); 7,58 (d, *J*_{HH}= 8,3 Hz, 1 H); 7,98-8,07 (m, 4H) ppm.
¹³C-NMR (CD₂Cl₂): 19,0; 19,2; 21,6; 21,7; 22,4; 22,6; 22,7; 28,1; 29,8; 73,3 (d, *J*_{CP}= 14,1 Hz); 120,2; 120,3; 121,1; 122,5; 123,2; 123,3; 124,5; 124,6; 125,1; 126,6; 126,7; 128,1; 128,6; 129,4; 129,9; 130,9; 131,1; 146,1; 146,6 (d, *J*_{CP}= 4,6 Hz) ppm.

### Ligand 1S

### 4-(Cyclododecyloxy)-S-dinaphtho[2,1-d:1',2'-f][1,3,2]dioxaphosphepin

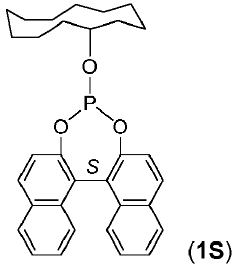

Zu einer bei 0 °C gerührten Mischung aus Cyclododecanol (0,489 g; 2,651 mmol), Toluol (14 ml) und Triethylamin (3,38 ml) wird eine Lösung von 4-Chlor-*S*-dinaphtho[2,1-*d*:1',2'-*f*][1,3,2]dioxaphosphepin (0,930 g; 2,651 mmol) in Toluol (6 ml) getropft. Man lässt auf Raumtemperatur kommen, rührt über Nacht, filtriert und engt das Filtrat im Vakuum bis zur Trockne ein. Der erhaltene Rückstand wird 2 h bei 50°C/0,1 mbar getrocknet und mittels Umkristallisation aus heißem Acetonitril gereinigt. Ausbeute: 0,893 g (1,791 mmol; 67%). Elementaranalyse (ber. für C₃₂H₃₅O₃P= 498,599 g/mol): C 77,12 (77,09); H 7,23 (7,07); P 6,08 (6,21)%.
ESI-TOF/HRMS: *m*/*e* 537,19654 (*M*+K)⁺.
³¹P-NMR (CD₂Cl₂): 150,2 (s) ppm.
¹H-NMR (CD₂Cl₂): 1,11-179 (m, 22H); 4,37 (m, 1H); 7,11-7,45 (m, 8H); 781-7,90 (m, 4H) ppm.
¹³C-NMR (CD₂Cl₂): 21,1; 21,3; 23,6; 23,7; 24,4; 24,7; 24,8; 31,8; 31,9; 75,4 (d, *J*_{CP}= 14,0 Hz); 122,2; 123,1; 123,3; 124,5; 124,6; 125,3; 125,4; 126,6; 126,7; 127,1; 128,7; 128,8; 130,2; 130,7; 131,5; 131,9; 133,0; 133,2; 148,2; 148,7 (d, *J*_{CP}= 5,0 Hz) ppm.

### Ligand 2

### 4,8-Di-tert-butyl-6-(cyclododecyloxy)-2,10-dimethoxydibenzo[d,f][1,3,2]dioxaphosphepin

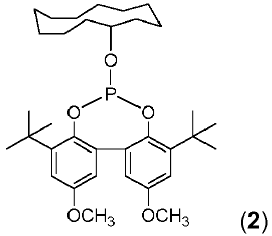

Zu einer auf -20 °C gekühlten Lösung von Cyclododecanol (0,298 g; 1,618 mmol) in THF (4 ml) tropft man unter Rühren eine 0,32 M Lösung von *n*-Butyllithium in Heptan (5,06 ml; 1,618 mmol). Nach Zugabe wird noch 20 min gerührt, man lässt auf Raumtemperatur kommen und setzt tropfenweise eine Lösung von 4,8-Di-*tert*-butyl-6-chloro-2,10-dimethoxydibenzo[*d,f*][1,3,2]dioxaphosphepin (0,684 g; 1,618 mmol) in THF (4 ml) zu. Man lässt über Nacht rühren, entfernt das Lösungsmittel im Vakuum, nimmt den Rückstand in Toluol (8 ml) auf, und filtriert. Das Filtrat wird im Vakuum entfernt, der sirupöse Rückstand zunächst 2 h bei 50°C/0,1 mbar getrocknet, dann in heißem Acetonitril (11 ml) aufgenommen. Man lässt langsam abkühlen und 3 Tage bei Raumtemperatur stehen. Der abgeschiedene Feststoff wird nochmals in gleicher Weise mit Acetonitril (9 ml) behandelt und das Produkt des zweiten Reinigungsschrittes abschließend im Vakuum (0,1 mbar) getrocknet. Ausbeute: 0,488 g (0,855 mmol; 53%). Elementaranalyse (ber. für C₃₄H₅₁O₅P= 570,746 g/mol): C 71,47 (71,55); H 9,06 (9,00); P 5,50 (5,43)%.
ESI-TOF/HRMS: m/e 609,31126 (*M*+K)⁺.
³¹P-NMR (CD₂Cl₂): 148,2 (s) ppm.
¹H-NMR (CD₂Cl₂): 1,30-1,88 (überlappende Signale, 40H); 3,85 (s, 6 H); 4,55 (m, 1H); 6,76 (d, *J*_{HH}= 3,1 Hz, 2H); 7,02 (d, *J*_{HH}= 3,1 Hz, 2H) ppm.
¹³C-NMR (CD₂Cl₂): 21,2; 23,6; 23,7; 24,4; 24,8; 31,2; 31,7; 35,7; 55,9; 75,0 (d, *J*_{CP}= 15 Hz); 113,2; 114,6; 134,2; 142,2 (d, *J*_{CP}= 6,4 Hz); 142,9; 156,0 ppm.

### Ligand 3

### 2,4,8,10-Tetra-tert-butyl-6-(cyclododecyloxy)dibenzo[d,f][1,3,2]dioxaphosphepin

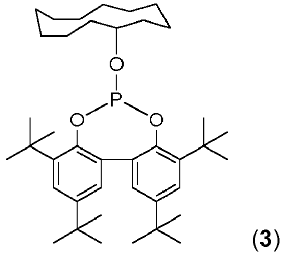

Zu einer auf 0 °C gekühlten Mischung aus Cylododecanol (0,236 g; 1,278 mmol), Triethylamin (1,63 ml) und Toluol (8 ml) wird unter Rühren eine Lösung von 2,4,8,10-Tetra-*tert*-butyl-6-chlorodibenzo[*d,f*][1,3,2]dioxaphosphepin (0,607 g; 1,278 mmol) in Toluol (3 ml) getropft. Man lässt über Nacht rühren, filtriert, entfernt das Lösungsmittel im Vakuum und trocknet den Rückstand 2 h bei 50°C/0,1 mbar. Das Rohprodukt wird aus heißem Acetonitril (10 ml) kristallisiert. Mehrtägige Lagerung im Kühlschrank ergibt 0,607 g (0,975 mmol; 76%). Elementaranalyse (ber. für C₄₀H₆₃O₃P= 622,908 g/mol): C 77,06 (77,13); H 10,16 (10,19); P 4,98 (4,97)%.
ESI-TOF/HRMS: m/e 661,41492 (*M*+K)⁺.
³¹P-NMR (CD₂Cl₂): 147,9 (s) ppm.
¹H-NMR (CD₂Cl₂): 1,36-1,89 (überlappende Signale, 58H); 4,57 (m, 1 H); 7,21 (d, *J*_{HH}= 2,5 Hz, 2H); 7,50 (d, *J*_{HH}= 2,5 Hz, 2H) ppm.
¹³C-NMR (CD₂Cl₂): 21,3: 23,6; 23,7; 24,3; 24,7; 31,4; 31,7; 35,0; 35,7; 75,1 (d, *J*_{CP}= 15,5 Hz); 124,7; 126,8; 133,3; 140,6; 146,2 (d, *J*_{CP}= 6,5 Hz); 146,9 ppm.

### Ligand 4

### a) Dichlor(cyclododecyloxy)phosphan (Vorstufe)

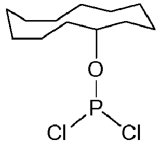

Zu einer bei 10 °C gerührten Lösung von Phosphortrichlorid (5,19 g; 37,8 mmol) in THF (25 ml) wird innerhalb von 90 min eine Mischung aus Cyclododecanol (1,935 g; 10,5 mmol), Triethylamin (2 ml) und THF (80 ml) getropft. Man lässt auf Raumtemperatur kommen, rührt über Nacht, filtriert, und entfernt flüchtige Anteile im Vakuum. Der Rückstand wird 5 h bei 50°C/0,1 mbar getrocknet. Ausbeute: 2,776 g (93%).
³¹P-NMR (CD₂Cl₂): 175,5 (s) ppm.
Das nach ³¹P- und ¹H-NMR-spektroskopischem Befund in einer Reinheit von 94 mol-% vorliegende Produkt wurde wie erhalten im nächsten Schritt eingesetzt.

### b) 9-(tert-Butyl)-6-(cyclododecyloxy)-4-methoxy-2-methyldibenzo[d,f][1,3,2]dioxaphosphepin (Ligand 5)

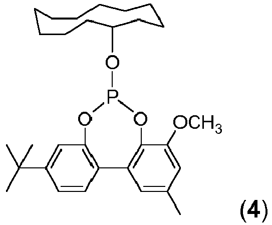

Zu einer auf 0 °C gekühlten Mischung aus 4'-(*tert*-Butyl)-3-methoxy-5-methyl-[1,1'-biphenyl]-2,2'-diol (0,582 g; 2,033 mmol), Triethylamin (2,6 ml) und Toluol (8 ml) wird unter Rühren eine Lösung von Dichlor(cyclododecyloxy)phosphan (0,580 g; 2,034 mmol) in Toluol (5 ml) getropft. Man lässt über Nacht bei Raumtemperatur rühren, filtriert, engt das Filtrat im Vakuum ein, trocknet den Rückstand bei 0,1 mbar und löst das erhaltene Rohprodukt in warmem Acetonitril. Lagerung bei -23 °C liefert nach 3 Tagen 0,879 g Feststoff (1,763 mmol; 87%).
Elementaranalyse (ber. für C₃₀H₄₃O₄P= 498,6397 g/mol): C 72,03 (72,26); H 8,89 (8,69); P 6,15 (6,21)%.
ESI-TOF/HRMS: *m*/*e* 521,27944 (*M*+Na)⁺.
³¹P-NMR (CD₂Cl₂): 149,1 (s) ppm.
¹H-NMR (CD₂Cl₂): 1,29-1,90 (Signalüberlappung, 27H); 1,70 (m, 2H), 1,87 (m, 2H); 2,44 (s, 3H), 3,93 (s, 3H); 4,60 (m, 1 H); 6,83 (m, 2H); 7,22 (m, 1 H); 7,35 (m, 1 H); 7,45 (m, 1 H) ppm.
¹³C-NMR (CD₂Cl₂): 20,9; 21,6; 23,4; 23,5; 23,6; 24,7; 25,0; 30,1; 31,4; 31,5; 31,6; 35,0; 56,2; 74,9 (d, *J*_{CP}= 14,0 Hz); 112,7; 119,4; 121,8; 122,4; 128,5; 129,6; 132,2; 135,2; 136,5; 149,8 (d, *J*_{CP}= 5,8 Hz); 151,7; 153,3 ppm.

### Ligand 5

### 6-(Cyclododecyloxy)dibenzo[d,f][1,3,2]dioxaphosphepin

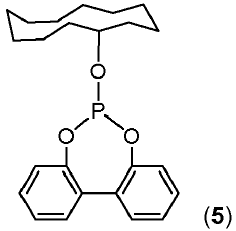

Zu einer bei 0 °C gerührten Mischung aus Cyclododecanol (0,495 g; 2,84 mmol), Toluol (15 ml) und Triethylamin (3,4 ml) wird eine Lösung von 6-chlorodibenzo[*d,f*][1,3,2]dioxaphosphepin (0,740 g; 2,952 mmol) in Toluol (6 ml) getropft. Man lässt auf Raumtemperatur kommen, rührt über Nacht, filtriert und engt das Filtrat im Vakuum bis zur Trockne ein. Der erhaltene viskose Rückstand wird 2 h bei 50°C/0,1 mbar getrocknet und säulenchromatografisch gereinigt (Hexan/Toluol=1:1, R*_{f}*= 0,5). Ausbeute: 0,810 g (2,03 mmol; 75%).
Elementaranalyse (ber. für C₂₄H₃₁O₃P= 398,4799 g/mol): C 72,49 (72,34); H 8,05 (7,84); P 7,70 (7,77)%.
ESI-TOF/HRMS: m/e 421,19066 (*M*+Na)⁺.
³¹P-NMR (CD₂Cl₂): 148,4 (s) ppm.
¹H-NMR (CD₂Cl₂): 1,31-1,61 (multiple Signalüberlappung, 16H); 1,69 (m, 2H); 1,89 (m, 2H); 4,61 (m, 1 H); 7,26 (m, 2H); 7,35 (m, 2H); 7,44 (m, 2H); 7,55 (m, 2H) ppm.
¹³C-NMR (CD₂Cl₂): 21,2; 23,6; 23,7; 24,5; 24,8; 31,8; 74,9 (d, *J*_{CP}= 13,5 Hz); 122,5; 125,5; 129,6; 130,3; 131,6 (d, *J*_{CP}= 3,3 Hz); 150,0 (d, *J*_{CP}= 5,2 Hz) ppm.

### Ligand 6

### 2-(Cyclododecyloxy)-4,4,5,5-tetraphenyl-1,3,2-dioxaphospholan

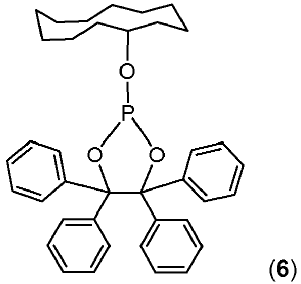

Zu einer bei -20 °C gerührten Lösung von Cyclododecanol (0,324 g; 1,757 mmol) in THF (4 ml) tropft man eine 0,533 M Lösung von n-Butyllithium (3,3 ml; 1,757 mmol), lässt zunächst weitere 20 min bei derselben Temperatur rühren und tropft dann bei Raumtemperatur eine Lösung von 2-Chlor-4,4,5,5-tetraphenyl-1,3,2-dioxaphospholan (0,757 g; 1,757 mmol) in THF (5 ml) zu. Man rührt über Nacht, filtriert, engt das Filtrat im Vakuum bis zur Trockne ein und nimmt den Rückstand in Toluol (10 ml) auf. Der erhaltene, zunächst viskose Rückstand wird bei 50°C/0,1 mbar getrocknet, und anschließend bei Raumtemperatur für 1 h mit Heptan (12 ml) verrührt. Man filtriert, und trocknet den erhaltenen Feststoff im Vakuum. Ausbeute: 0,553 g (0,955 mmol; 54%). Elementaranalyse (ber. für C₃₈H₄₃O₃P= 578,7287 g/mol): C 78,70 (78,87); H 7,40 (7,49); P 5,47 (5,35)%.
ESI-TOF/HRMS: *m*/*e* 601,28429 (*M*+Na)⁺.
³¹P-NMR (CD₂Cl₂): 148,0 (s) ppm.
¹H-NMR (CD₂Cl₂): 1,16-1,55 (Signalüberlappung, 22H); 4,32 (m, 1H); 7,04-7,23 (m, 16H); 7,50 (m, 4H) ppm.
¹³C-NMR (CD₂Cl₂): 21,1; 23,6; 23,7; 24,4; 24,7; 31,7; 73,0 (d, *J*_{CP}= 23,0 Hz); 127,2; 127,4; 129,2; 130,3; 142,9; 143,0; 143,4 ppm.

### Ligand 7 (Vergleichsbeispiel)

### 6-([1,1':3',1"-Terphenyl]-2'-yloxy)-4,8-di-tert-butyl-2,10-dimethoxydibenzo[d,f][1,3,2]dioxaphosphepin

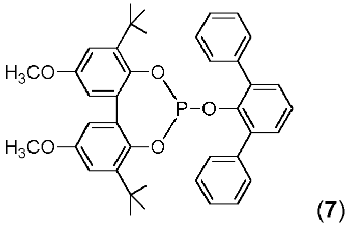

Eine Lösung von 2,6-Diphenylphenol (0,411 g; 1,65 mmol) in Toluol (8 ml) wurde mit Triethylamin (1,529 g; 15,11 mmol) versetzt und die erhaltene Mischung tropfenweise bei 0 °C zu einer Lösung von 4,8-Di-*tert*-butyl-6-chloro-2,10-dimethoxydibenzo [*d,f*][1,3,2]dioxaphosphepin (0,697 g; 1,65 mmol) in Toluol (6 ml) gegeben. Die Reaktionsmischung wurde über Nacht bei Raumtemperatur und für 5 h bei 70 °C gerührt. Dann wurde die Mischung filtriert und das Filtrat im Vakuum bis zur Trockne eingeengt. Der erhaltene Rückstand wurde aus Hexan (4 ml) umkristallisiert. Ausbeute: 0,417 g (0,659 mmol; 40 %). Elementaranalyse (ber. für C₄₀H₄₁O₅P = 632,70 g/ mol) C 75,95 (75,93); H 6,52 (6,53); P 5,01 (5,00) %.
³¹P-NMR (CD₂Cl₂): 140,9 ppm.
¹H-NMR (CD₂Cl₂): 1,37 (s, 18 H); 3,84 (s, 6 H); 6,51 (d, ⁵*J*_{HH}= 3,1 Hz, 2 H, Hₐᵣₒₘ); 6,89-7,95 (m, br, 15 H, Hₐᵣₒₘ) ppm.
¹³C-NMR (CD₂Cl₂): 31,1; 35,5; 55,9; 113,0; 114,5; 125,2; 126,5-131,0; 133,8 (d, *J*_{CP}= 4,0 Hz); 141,5 (d, *J*_{CP}= 6,3 Hz); 142,6; 144,8; 156,1 ppm.
ESI-TOF/HRMS: m/e 633,27654 (M+H)⁺.

### Ligand 8 (Vergleichsbeispiel)

### 6-(Anthracen-9-yloxy)-4,8-di-tert-butyl-2,10-dimethoxydibenzo[d,f][1,3,2]dioxaphosphepin

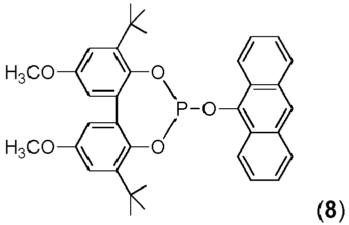

Eine gerührte Lösung von Anthracen-9-ol (0,388 g; 2 mmol) in Toluol (9 ml) wurde mit Triethylamin (1,854 g; 18,33 mmol) versetzt und die erhaltene Mischung tropfenweise bei 0 °C zu einer Lösung von 4,8-Di-*tert*-butyl-6-chloro-2,10-dimethoxydibenzo [*d,f*][1,3,2]dioxaphosphepin (0,845 g; 2 mmol) in Toluol (8 ml) gegeben. Die Reaktionsmischung wurde über Nacht gerührt und filtriert. Das Filtrat wird im Vakuum bis zur Trockne eingeengt. Der erhaltene Rückstand wurde zweimal mit Hexan (je 4 ml) verrührt und filtriert, und dann im Vakuum getrocknet. Ausbeute: 0,206 g (0,355 mmol; 18 %).
Elementaranalyse (ber. für C₃₆H₃₇O₅P = 580,63 g/ mol) C 74,28 (74,46); H 6,62 (6,42); P 5,39 (5,33) %.
³¹P-NMR (CD₂Cl₂): 140,4 ppm.
¹H-NMR (CD₂Cl₂): 1,39 (s, 18 H); 3,92 (s, 6 H); 6,93 (d, ⁵*J*_{HH}= 3,1 Hz, 2 H, Hₐᵣₒₘ); 7,14 (d, ⁵*J*_{HH}= 3,1 Hz, 2 H, Hₐᵣₒₘ); 7,51 (m, 4 H, Hₐᵣₒₘ); 8,05 (m, 2 H, Hₐᵣₒₘ); 8,34 (s, 1 H, Hₐᵣₒₘ); 8,41 (m, 2 H, Hₐᵣₒₘ) ppm.
¹³C-NMR (CD₂Cl₂): 31,0; 35,7; 56,0; 113,5; 114,9; 123,2; 123,7 (d, *J*_{CP}= 5,4 Hz); 124,8; 125,9; 126,0; 128,4; 132,6; 134,2 (d, *J*_{CP}= 3,9 Hz); 141,7 (d, *J*_{CP}= 6,0 Hz); 143,4; 143,5; 156,6 ppm.
ESI-TOF/HRMS: m/e 581,24490 (M+H)⁺.

### Ligand 9 (Vergleichsbeispiel)

### 6-(Anthracen-9-yloxy)-2,4,8,10-tetra-tert-butyldibenzo[d,f][1,3,2]dioxaphosphepin.

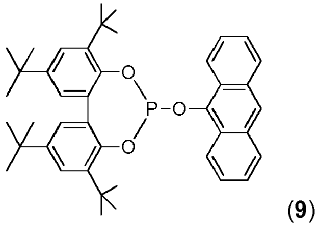

Eine gerührte Suspension von Anthracen-9-ol (0,281 g; 1,45 mmol) in Toluol (7 ml) wurde mit Triethylamin (1,344 g; 13,28 mmol) versetzt und die erhaltene Mischung tropfenweise bei 0 °C zu einer Lösung von 2,4,8,10-Tetra-*tert*-butyl-6-chlorodibenzo [*d,f*][1,3,2]dioxaphosphepin (0,724 g; 1,52 mmol) in Toluol (6 ml) gegeben. Die Reaktionsmischung wurde über Nacht gerührt und filtriert. Das Filtrat wurde im Vakuum bis zur Trockne eingeengt und der erhaltene Rückstand aus Hexan (4 ml) umkristallisiert. Ausbeute: 0,559 g (0,883 mmol; 61 %).
Elementaranalyse (ber. für C₄₂H₄₉O₃P = 632,78 g/ mol) C 79,83 (79,71); H 7,61 (7,81); P 5,01 (4,89) %.
³¹P-NMR (CD₂Cl₂): 140,7 ppm.
¹H-NMR (CD₂Cl₂): 1,41 (s, 18 H, C(C*H*₃)₃); 1,50 (s, 18 H, C(C*H*₃)₃); 7,40 (d, ⁵*J*_{HH}= 2,4 Hz, 2 H, Hₐᵣₒₘ); 7,43-7,56 (m, 4 H, Hₐᵣₒₘ); 7,60 (d, ⁵*J*_{HH}= 2,4 Hz, 2 H, Hₐᵣₒₘ); 8,05 (m, 2 H, Hₐᵣₒₘ); 8,34 (s, 1 H, Hₐᵣₒₘ); 8,38 (m, 2 H, Hₐᵣₒₘ) ppm.
¹³C-NMR (CD₂Cl₂): 31,2; 31,2; 31,8; 35,1; 35,7; 123,1; 123,7 (d, *J*_{CP}= 5,3 Hz); 124,8; 125,0; 125,8; 126,0; 127,2; 128,4; 132,6; 133,3 (d, *J*_{CP}= 3,7 Hz); 141,0; 143,5; 145,8 (d, *J*_{CP}= 6,2 Hz); 147,7 ppm.
ESI-TOF/HRMS: m/e 633,34934 (M+H)⁺.

### Durchführung der Katalyseversuche

Die Hydroformylierung wurde in mit Druckkonstanthaltung, Gasflussmessung, Begasungsrührer und Druckpipette ausgestattete 200 ml-Autoklaven der Fa. Premex Reactor AG, Lengau, Schweiz, durchgeführt. Das als Lösungsmittel verwendete Toluol wurde mit einem Pure Solv MD-7 System gereinigt und unter Argon aufbewahrt. Das als Substrat eingesetzte Substrat n-Octene (Octenisomerengemisch aus 1-Octen: ∼3 %; *cis*+*trans*-2-Octen: 48 %; *cis*+*trans*-3-Octen*:* 29%; *cis*+*trans*-Octen-4: 17 %; gerüstisomere Octene: 3 %) wurde mehrere Stunden über Natrium am Rückfluß erhitzt und unter Argon destilliert.

Für die Versuche wurden im Autoklaven unter Argonatmosphäre Lösungen der Katalysatorvorstufe und des Liganden gemischt. Als Katalysatorvorstufe kam [(acac)Rh(COD)] (Umicore, acac=Acetylacetonat-Anion; COD = 1,5-Cyclooctadien) zum Einsatz. Für Versuche mit einer Konzentration von 100 ppm-m Rhodium wurden 10 ml einer 4,31 millimolaren Lösung in den Autoklaven gegeben. Anschließend wurde die einem Verhältnis L/Rh = 5:1 entsprechende Masse des Liganden in 10 ml Toluol gelöst und zugemischt. Durch Zugabe von weiterem Toluol wurde das Anfangsvolumen der Katalysatorlösung auf 41,0 ml eingestellt. In eine druckfeste Pipette wurde eingefüllt: n-Octene (10,70 g; 95,35 mmol). Der Autoklav wurde mit Synthesegas (Linde; H₂ (Qualität 5.0): CO (Qualität 4.7) = 1:1) auf einen Gesamtdruck von 42 bar gebracht und auf 120°C aufgeheizt. Nach Erreichen der Reaktionstemperatur wurde der Synthesegasdruck im Autoklaven auf 48,5 bar erhöht und das Olefingemisch mit einem in der Druckpipette eingestellten Druck von ca. 51,5 bar in den Autoklaven gepresst. Die Reaktion wurde bei konstantem Druck von 50 bar (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm.

Die Ergebnisse sind in der nachfolgenden Tabelle gezeigt.

| **Ligand** | **Ausbeute [%]** | **Selektivität [%]** |
|---|---|---|
| 1* | 98 | 6,3 |
| 1S* | 96 | 6,3 |
| 2* | 97 | 15,7 |
| 3* | 99 | 14,9 |
| 4* | 99 | 5,0 |
| 5* | 98 | 5,7 |
| 6* | 99 | 11,3 |
| Alkanox 240 | 95 | 20 |
| 7 | 93 | 28,8 |
| 8 | 97 | 27,7 |
| 9 | 98 | 26,1 |

| | | |
|---|---|---|
| * erfindungsgemäße Verbindungen Selektivität % = n / (n + i) | | |

Alkanox 240 wurde unter identischen Bedingungen gemessen. Bei Alkanox 240 handelt es sich um einen aus dem Stand der Technik bekannten Triphosphitliganden Tris(2,4-di-tert-butylphenyl)phosphit.

Die Ergebnisse zeigen, dass die erfindungsgemäßen Liganden ähnlich gute Ausbeuten erzielen wie Alkanox 240, allerdings bei einer deutlich geringeren n/i-Selektivität. Somit ist es möglich, Gemische von n/iso-Aldehyden herzustellen.

Die Vergleichsverbindungen 7 bis 9 weisen, trotz ihrer großen strukturellen Ähnlichkeit zu den Liganden 2 und 3, eine erhöhte n/i-Selektivität auf. Dies demonstriert, dass durch Auswahl eines Cyclodocanol-Substituenten gemäß Formel (I) die n/i-Selektivität minimiert werden kann.

## Patentansprüche

1. Verbindung gemäß Formel (I) 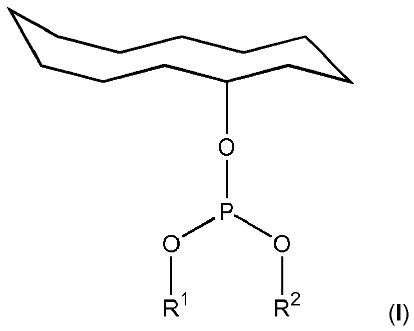 wobei R¹ und R² jeweils eine Cyclododecanylgruppe oder zwei kovalent miteinander verknüpfte (C₆-C₂₀)-Arylgruppen darstellen oder zusammen eine (C₆-C₂₀)-Arylgruppe oder eine (C₁-C₁₂)-Alkylgruppe bilden,
wobei R¹ und R² mit einem oder mehreren Substituenten ausgewählt aus der Gruppe bestehend aus -H, -(C₆-C₂₀)-Aryl, -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl,-CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -COOH, -OH, -SO₃H, -NH₂, und Halogen substituiert sein können.

2. Verbindung nach Anspruch 1,
wobei R¹ und R² jeweils eine Cyclododecanylgruppe oder zwei kovalent miteinander verknüpfte Phenyl- oder Naphthylgruppen dar oder bilden zusammen eine Phenyl- oder eine Ethylgruppe, wobei R¹ und R² mit einem oder mehreren Substituenten ausgewählt aus der Gruppe bestehend aus -H, -(C₆-C₂₀)-Aryl, -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl,-CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -COOH, -OH, -SO₃H, -NH₂, und Halogen substituiert sein können.

3. Verbindung nach einem der Ansprüche 1 bis 2,
wobei die Verbindung ausgewählt ist aus einer der Strukturen gemäß den Formeln **(II)** bis **(VI):** 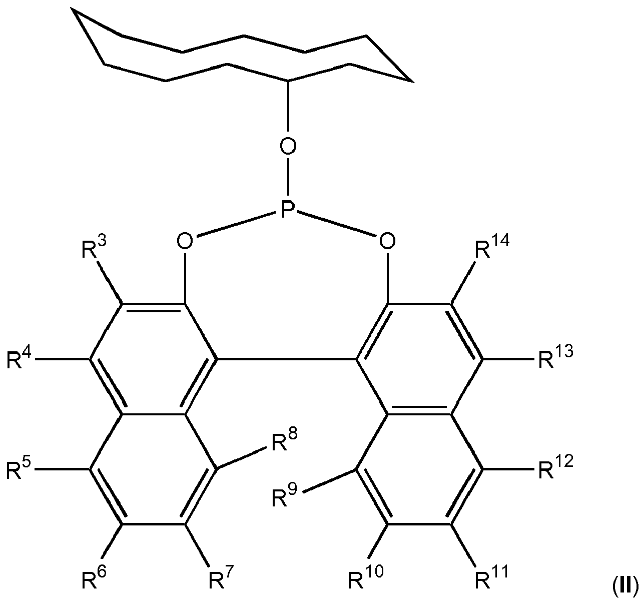 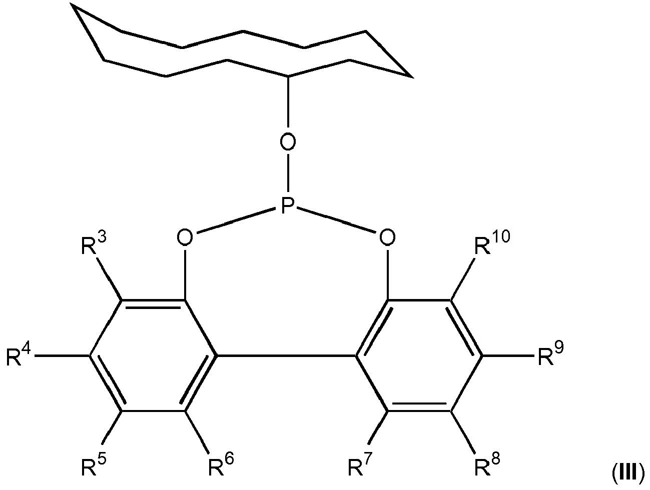 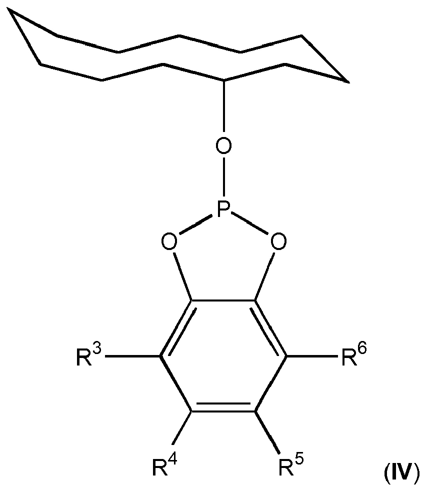 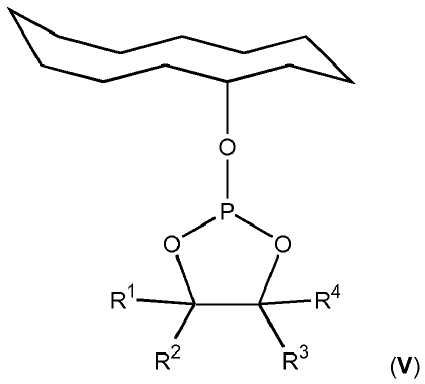 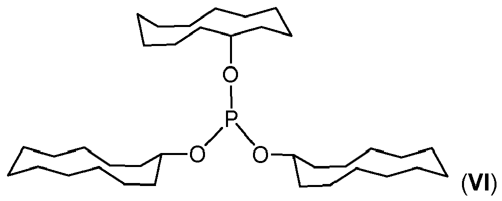 wobei R³ bis R¹⁴ unabhängig voneinander ausgewählt sind aus der Gruppe bestehend aus -H, -(C₆-C₂₀)-Aryl, -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl,-CO-(C₁-C₁₂)-Alkyl, -CO-(C₃-C₁₂)-Cycloalkyl, -N-[(C₁-C₁₂)-Alkyl]₂, -COOH, -OH, -SO₃H,-NH₂, und Halogen.

4. Verbindung nach Anspruch 3,
wobei R³ bis R¹⁴ unabhängig voneinander ausgewählt sind aus der Gruppe bestehend aus -H, -Phenyl, -(C₁-C₆)-Alkyl, und -O-(C₁-C₆)-Alkyl.

5. Verbindung nach einem der Ansprüche 1 bis 4,
ausgewählt aus einer der Strukturen gemäß den Formeln (1) bis (6), wobei von Formel (1) auch die reinen S- und R-Enantiomere umfasst sind:

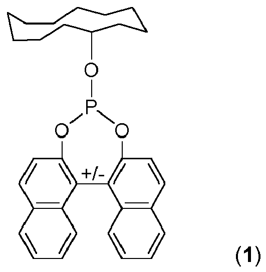

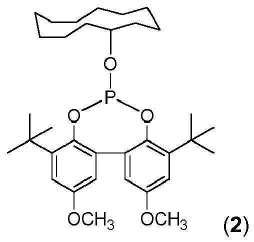

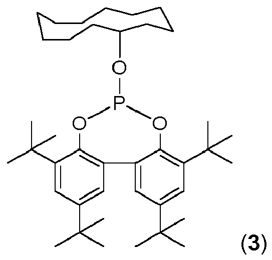

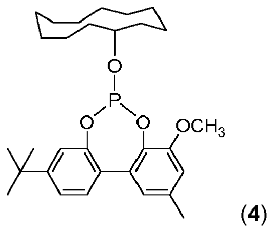

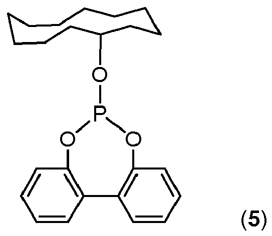

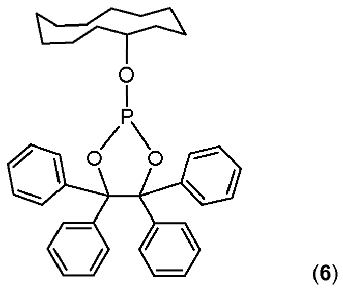

6. Komplex umfassend eine Verbindung nach einem der Ansprüche 1 bis 5 und ein Metallatom ausgewählt aus Rh, Ru, Co und Ir.

7. Verwendung einer Verbindung nach einem der Ansprüche 1 bis 5 zur Katalyse einer Hydroformylierungsreaktion.

8. Verwendung eines Komplexes nach Anspruch 6 zur Katalyse einer Hydroformylierungsreaktion.

9. Verfahren zur Herstellung eines Aldehyds, umfassend die Verfahrensschritte:
a) Vorlegen eines Olefins,
b) Zugabe eines Komplexes nach Anspruch 6 oder einer Verbindung nach einem der Ansprüche 1 bis 5 und einer Substanz, welche ein Metallatom ausgewählt aus Rh, Ru, Co und Ir aufweist,
c) Zuführen von Wasserstoff und Kohlenstoffmonoxid,
d) Erwärmen des Reaktionsgemisches, wobei das Olefin zu einem Aldehyd umgesetzt wird.

10. Verfahren nach Anspruch 9,
wobei das molare Verhältnis der Verbindung nach einem der Ansprüche 1 bis 5 zu dem Metallatom nach Anspruch 6 im Bereich von 10:1 bis 1:1 liegt.

11. Verfahren nach einem der Ansprüche 9 und 10,
wobei die Umsetzung des Olefins zu einem Aldehyd bei einem Druck im Bereich von 30 bar bis 70 bar stattfindet.
